# DEMANDE DE BREVET EUROPEEN

(11) **EP 0 752 238 A1**
(43) Date de publication de la demande: **08.01.1997**
(21) Numéro de dépôt: 95420184.4
(22) Date de dépôt: 03.07.1995
(51) Int. Cl.: A61B 19/02

(54) **Procédé et dispositif de conditionnement d'une pièce de liaison pour éléments osseux**

(71) Demandeur: Bertholet, Maurice, F-38360 Sassenage (FR)
(72) Inventeur: Bertholet, Maurice, F-38360 Sassenage (FR)
(74) Mandataire: Chabrelie, André

(57) **Abrégé**

Un dispositif pour la liaison d'éléments comprend :
une pièce de liaison (1) comprenant une partie centrale et au moins deux parties d'ancrage (2, 3) liées rigidement à la partie centrale, au moins une partie de la pièce étant réalisée en un matériau à mémoire de forme ; et
un appareil de conditionnement comprenant un support (5) incluant des logements (7, 8) adaptés pour recevoir respectivement lesdites parties d'ancrage (3, 4) lorsque ces dites parties d'ancrage sont dans des positions d'éloignement maximum les unes par rapport aux autres.

## Description

### Domaine de l'invention

La présente invention concerne de façon générale des pièces de liaison pour des éléments osseux, ces pièces de liaison comprenant généralement une partie centrale et au moins deux parties d'ancrage disposées sensiblement au voisinage d'extrémités de la partie centrale. La présente invention concerne aussi des appareils de conditionnement pour de telles pièces de liaison.

### Commentaires de l'art antérieur

On connaît un grand nombre de pièces de liaison destinées à relier ensemble des éléments osseux, afin de faciliter la reconstitution osseuse entre ces éléments osseux.

Par exemple au travers du document CA-A-1 149 106, il est déjà connu de constituer des agrafes ou des plaques chirurgicales pour la fixation des os et des tissus mous, dans lesquelles une partie centrale (réf. 15 dans ce document) de la plaque comprend un trou ou une fenêtre qui définit deux parties latérales (réf. 10 et 11 dans ce document) en un métal relativement rigide mais déformable non plastiquement. Dans l'état initial (lors de la fixation sur l'os par le chirurgien), les parties latérales (réf. 10 et 11 dans ce document) sont sensiblement rectilignes, formant ainsi une fenêtre sensiblement rectangulaire. Après la phase initiale de fixation sur l'os, le chirurgien, celui-ci prend une pince et serre les la plaque entre les parties latérales rectilignes (réf. 10 et 11 dans ce document), ce qui produit une déformation plastique sous forme d'une incurvation de ces parties latérales en faisant en sorte que leur zones centrales se rapprochent l'une de l'autre (comme on peut le voir en traits pleins sur la figure 1, réf. 10 et 11, dans ce document).

Un inconvénient d'un tel dispositif connu est le suivant. Un matériau métallique relativement rigide mais pouvant se déformer plastiquement ne peut jamais se déformer uniquement plastiquement mais se déforme à la fois plastiquement et élastiquement. Par conséquent, lorsque le chirurgien resserre à l'aide d'une pince les parties latérales pour les rapprocher l'une de l'autre plastiquement, suite au relâchement du serrage à l'aide de la pince, les parties latérales reviennent élastiquement légèrement en direction de leur position initiale. Ce léger retour élastique a pour conséquence que les griffes d'extrémité (réf. 5 et 6 dans ce document) s'éloignent l'une de l'autre du fait de ce retour élastique et provoquent ainsi finalement un léger jeu entre les parties d'os reliées respectivement à l'une et l'autre de ces griffes, ce qui est désavantageux. En outre, la force de serrage produite par le chirurgien à l'aide le la pince ne peut pas être contrôlée. Enfin, les deux parties d'os raccordées ne sont pas en définitive soumises de façon permanente à une force élastique de rapprochement.

Par exemple au travers du document EP-A-0 488 906, il est déjà connu de constituer des agrafes ou des plaques chirurgicales pour la fixation des os et des tissus mous, dans lesquelles au moins une partie du matériau qui constitue l'agrafe ou la plaque est réalisée en un matériau à mémoire de forme. Un matériau à mémoire de forme est un matériau changeant de forme lorsqu'il passe d'une température inférieure à la température de transformation martensitique à une température supérieure à la température de transformation austénitique.

Il est également connu de constituer des agrafes ou des plaques d'ostéosynthèse réalisées en un alliage martensitique thermo-élastique, cette plaque ou cette agrafe étant éduquée pour se présenter sous une forme rectiligne à une température inférieure à la température de transformation martensitique du matériau qui la compose et sous une forme ondulée induisant un raccourcissement de sa longueur à une température supérieure à la température de transformation austénitique dudit matériau.

Un inconvénient des dispositifs de l'art antérieur réside dans le fait qu'un chirurgien ou une autre personne présente dans un bloc opératoire doit intervenir manuellement sur l'agrafe afin de la déformer tandis que celle-ci est froide. Par exemple, cette personne extrait d'un compartiment réfrigéré une agrafe puis elle utilise des pinces pour écarter les deux pattes de l'agrafe. L'opération doit être effectuée rapidement parce que l'agrafe se réchauffe rapidement. Ensuite, cette personne doit amener l'agrafe froide ainsi déformée jusqu'en son lieu d'implantation dans l'os du patient. Si les pattes de l'agrafe ont été écartées trop ou pas assez, le chirurgien ne peut pas correctement implanter l'agrafe dans l'os du patient. Si des interventions manuelles successives sont effectuées afin de corriger exactement l'écartement des pattes de l'agrafe, le temps mis pour effectuer cela devient suffisant pour réchauffer l'agrafe et la rendre inopérante pour subir la déformation plastique souhaitée.

D'autre part, de telles interventions manuelles sur une agrafe doivent être opérées tandis que l'agrafe reste parfaitement stérile, ce qui complique les opérations manuelles.

D'autre part, le lieu de stockage dans un compartiment réfrigéré est souvent situé à une grande distance du lieu même de l'intervention chirurgicale, ce qui oblige le personnel présent dans le bloc opératoire à effectuer des déplacements entre le compartiment réfrigéré dans lequel sont stockées les agrafes et le lieu même de l'intervention chirurgicale.

Un objet de la présente invention consiste à proposer un dispositif de conditionnement d'une pièce de liaison pour éléments osseux utilisant un matériau à mémoire de forme, ce dispositif de conditionnement étant destiné à simplifier les interventions manuelles sur la pièce de liaison par le personnel présent dans un bloc opératoire.

Un autre objet de la présente invention consiste à proposer un tel dispositif de conditionnement permettant en outre d'améliorer les conditions de stérilité des pièces de liaison utilisées.

Un autre objet de la présente invention consiste à proposer un tel dispositif de conditionnement permettant en outre d'améliorer les diverses opérations manuelle et de gestion lors du stockage à long terme des pièces de liaison, lors de leur transport jusque dans un bloc opératoire et lors de leur utilisation dans le bloc opératoire.

### Résumé de l'invention

L'invention concerne donc un dispositif pour la liaison d'éléments osseux

Selon une caractéristique essentielle de l'invention, le dispositif comprend une pièce de liaison comprenant une partie centrale et au moins deux parties d'ancrage liées rigidement à la partie centrale, au moins une partie de la pièce étant réalisée en un matériau à mémoire de forme ; et un appareil de conditionnement comprenant un support incluant des logements adaptés pour recevoir respectivement lesdites parties d'ancrage lorsque ces dites parties d'ancrage sont dans des positions d'éloignement maximum les unes par rapport aux autres.

Selon un mode de réalisation de l'invention, ladite partie centrale présente une forme générale d'un anneau circulaire ou ovale.

Selon un autre mode de réalisation de l'invention, ladite pièce de liaison est une plaque de genou comprenant une partie centrale et au moins deux parties d'ancrage.

Selon un autre mode de réalisation de l'invention, ledit appareil de conditionnement comprend en outre un réservoir renfermant de façon étanche un produit de chaleur massique élevée de telle sorte qu'après que l'appareil de conditionnement a été refroidi puis placé en attente d'utilisation chirurgicale en un lieu à température ambiante, sa température reste basse pendant un temps relativement long, suffisant pour permettre à un chirurgien de choisir librement au cours d'une opération l'instant où il souhaite effectivement prélever la pièce de liaison placée dans l'appareil de conditionnement.

### Brève description des figures

Les figures 1A à 1H représentent respectivement diverses étapes successives d'un procédé de conditionnement d'une pièce de liaison pour éléments osseux à matériau à mémoire de forme selon la présente invention ;
la figure 2 représente en vue de côté un mode de réalisation d'une pince de saisie qui constitue l'une des pièces d'un dispositif de conditionnement selon la présente invention ;
les figures 3 et 4 représentent en coupe longitudinale une variante de réalisation de la pièce de saisie de la figure 2, la pièce de saisie étant représentée en position de serrage sur la figure 3 et en position de desserrage sur la figure 4 ;
la figure 5 est une vue en perspective représentant une variante de réalisation d'une pièce de liaison montée sur le dispositif de conditionnement selon la présente invention ;
les figures 6 et 7 représentent en vue de dessus schématique la pièce de liaison de la figure 5, respectivement en position étendue (figure 6) et en position rétractée (figure 7)
la figure 8 représente une agrafe de genou qui peut constituer un autre exemple d'utilisation d'une pièce de liaison pouvant être conditionnée à l'aide du dispositif de conditionnement selon la présente invention ; et
la figure 9 représente une agrafe à boutonnière qui peut constituer un autre exemple d'utilisation d'une pièce de liaison pouvant être conditionnée à l'aide du dispositif de conditionnement selon la présente invention.

### Description détaillée de l'invention

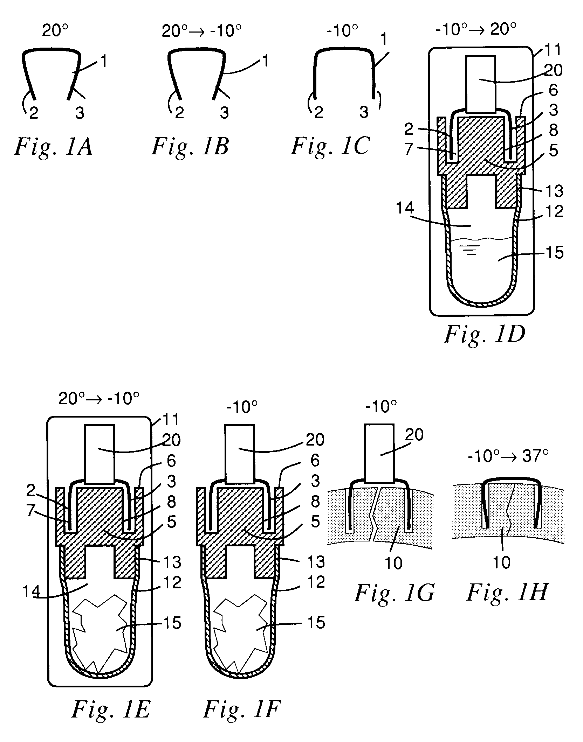

Si l'on se reporte aux figures 1A à 1H, on peut suivre les différentes étapes du procédé de conditionnement selon la présente invention. La première étape (figure 1A) consiste à agencer une pièce de liaison à matériau à mémoire de forme quelconque, par exemple, comme cela est représenté ici, une agrafe 1. Dans la réalité, un grand nombre d'agrafes identiques 1 sont fabriquées. L'agrafe 1 est à température ambiante et, dans cette condition, ses deux pattes 2, 3 ont tendance à se rapprocher l'une de l'autre, comme cela est représenté.

L'étape suivante (figure 1B) consiste à refroidir l'agrafe 1 jusqu'à une température inférieure à la température de transformation martensitique, par exemple jusqu'à 0 degré ou jusqu'à moins 10 degrés, comme cela est indiqué sur la figure.

L'étape suivante (figure 1C) consiste à déformer plastiquement l'agrafe 1 tandis qu'elle est maintenue à sa température basse. Dans l'exemple représenté, cette déformation consiste à écarter les deux pattes 2, 3 de l'agrafe 1 jusqu'à les amener par exemple parallèles l'une à l'autre.

L'étape suivante (figure 1D) consiste à réaliser un dispositif de conditionnement particulier, selon la présente invention, indiqué de façon générale par la référence 4. Ce dispositif de conditionnement 4 est essentiellement constitué par une pièce de support 5 comprenant une face supérieure 6 au niveau de laquelle débouchent un certain nombre de logements 7, 8 dont chacun est destiné à recevoir un élément de préhension correspondant 2, 3 de la pièce de liaison 1. Dans l'exemple qui est représenté ici, la pièce de support 5 comprend deux logements 7, 8 destinés à recevoir respectivement les pattes 2 et 3 de l'agrafe 1. Lorsque l'agrafe 1 est ainsi montée sur la pièce de support 5 tandis que ses pattes 2, 3 sont enfilées dans les logements 7, 8 correspondants, l'ensemble ainsi formé peut être ramené à la température ambiante. Le dispositif de conditionnement 4 sera décrit plus en détail par la suite.

L'étape suivante (aucune figure particulière) consiste à bloquer en longue durée le dispositif de conditionnement 4 ainsi muni de sa pièce de liaison 1. Pendant ce stockage de longue durée, la pièce de liaison 1 garde ses pattes 2, 3 écartées parce qu'elles sont maintenues fermement dans les logements correspondants 7, 8 de la pièce de support 5.

L'étape suivante (figure 1E) consiste à amener le dispositif de conditionnement 4 incluant la pièce de liaison 1 jusqu'en un lieu de stockage réfrigéré situé dans ou à proximité d'un bloc opératoire. Le dispositif 4 est alors refroidi jusqu'à une température inférieure à la température de transformation martensitique, par exemple jusqu'à 0 degré ou jusqu'à moins 10 degrés, comme cela est indiqué sur la figure. En général, on dispose ainsi d'un grand nombre de dispositifs de conditionnement 4 semblables dans un compartiment réfrigéré Chaque dispositif de conditionnement 4 inclut une pièce de liaison particulière, de telle sorte que des pièces de liaison 1 de différentes formes ou de différentes caractéristiques peuvent ainsi être stockées dans le compartiment réfrigéré situé dans ou à proximité du bloc opératoire. Le dispositif de conditionnement inclut une marque ou une indication servant de référence du type de pièce de liaison qu'il renferme.

L'étape suivante (figure 1F) consiste à extraire l'un des dispositifs de conditionnement 4 situés dans le compartiment réfrigéré en choisissant le dispositif de conditionnement 4 qui inclut la pièce de liaison 1 que l'on souhaite présentement utiliser dans le bloc opératoire. Le dispositif de conditionnement 4 ainsi saisi peut rester un long moment à la température ambiante dans le bloc opératoire parce que sa pièce de support 5 est réalisée d'une façon suffisamment massive pour rester froide pendant un temps relativement long, par exemple une demi-heure. Il résulte de cela que le compartiment réfrigéré servant à stocker des dispositifs de conditionnement 4 peut être situé à une relativement grande distance par rapport au bloc opératoire. En particulier, il n'est pas nécessaire que ce compartiment réfrigéré soit situé à l'intérieur même du bloc opératoire ou soit situé très près du lieu de l'intervention chirurgicale. A cet instant, le dispositif de conditionnement 4 ainsi extrait du compartiment réfrigéré peut être amené très près du lieu de l'intervention chirurgicale et peut être stocké ainsi un temps relativement long, par exemple une demi-heure. Le chirurgien peut saisir alors la pièce de liaison 1 de ce dispositif de conditionnement amené à proximité de lui, et il extrait la pièce de liaison 1 du dispositif 4 puis l'amène directement en place sur l'élément osseux 10 correspondant. La durée entre l'instant d'extraction de la pièce de liaison 1 de la pièce de support 5 et l'instant de la mise en place de la pièce de liaison 1 dans l'élément osseux 10 est extrêmement court parce qu'il n'est pas nécessaire entre ses deux instants d'intervenir manuellement sur la pièce de liaison pour la déformer plastiquement puisque cette déformation plastique est maintenue initialement grâce au fait que la pièce de liaison 1 est insérée dans des logements correspondants 7, 8 de la pièce de support 5. Cette étape d'insertion de la pièce de liaison dans l'élément osseux 10 correspond à la figure 1G.

Enfin, la dernière étape (figure 1H) consiste à laisser naturellement la pièce de liaison 1 insérée dans l'élément osseux 10 se réchauffer jusqu'à la température du corps du patient, ce qui provoque la déformation des éléments de préhension 2, 3 de la pièce de liaison 1, cette déformation permettant de créer dans l'élément osseux 10 une certaine force de compression et permettant en outre à la pièce de liaison 1 d'être maintenue en place d'une façon définitive.

L'invention concerne aussi la constitution et les fonctions du dispositif de conditionnement 4 qui a été décrit sommairement précédemment lors de la description du procédé selon l'invention.

Par conséquent, le dispositif de conditionnement 4, qui constitue un autre aspect de la présente invention, va être décrit maintenant en se reportant aux figures 1D et 1E précédemment évoquées lors de la description du procédé. Le dispositif de conditionnement 4 comporte, comme cela a déjà été décrit, tout d'abord, une pièce de support 5 qui comprend une face supérieure 6, par exemple une face plane, au niveau de laquelle débouchent un certain nombre de logements 7, 8 dont chacun est destiné à recevoir un élément de préhension 2, 3 d'une pièce de liaison pour éléments osseux à matériau à mémoire de forme 1. Le dispositif de conditionnement 4 comporte en outre une enveloppe externe étanche 11 qui est destinée à maintenir l'ensemble qu'elle renferme dans une condition stérile. L'enveloppe étanche 11 peut être réalisée par exemple sous la forme d'une poche en film plastique souple scellée. L'enveloppe externe 11 pourrait être également réalisée sous la forme d'une boite étanche réutilisable. Comme cela est représenté, il est en outre possible de prévoir dans la partie inférieure de la pièce de support 5 un réservoir 12 relié de façon étanche à la pièce de support 5 au niveau d'une partie de liaison étanche 13 et constituant une chambre interne étanche 14 renfermant un corps présentant une chaleur massique élevée 15 qui peut par exemple être de l'eau. Le corps 15 contribue à maintenir pendant un temps relativement long l'ensemble du dispositif de conditionnement 4 à une basse température lorsque ce dispositif est extrait du compartiment réfrigéré dans lequel il est habituellement stocké, afin d'être maintenu pendant un certain temps en attente d'une utilisation imminente par un chirurgien (comme cela a été décrit lors de l'étape du procédé illustrée par la figure 1F).

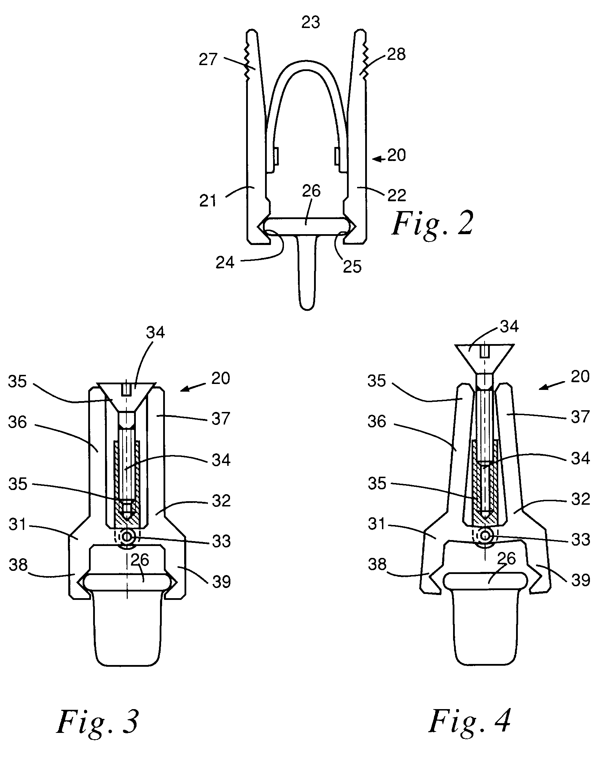

Comme on peut le voir sur les figures 1D à 1G, le dispositif de conditionnement 4 peut inclure en outre une pince spécifique 20 montée à demeure sur une partie externe de la pièce de liaison 1. Cette pince spécifique 20 est représentée plus en détail sur la figure 2. Sur la figure 2, on voit que la pince spécifique 20 comprend deux branches symétriques opposées 21, 22 reliées par un ressort 23 formé par une lame incurvée en forme de U. Les extrémités avant des branches 21, 22 comprennent des logements de réception 24, 25 dirigés vers l'intérieur et destinés à recevoir deux parties opposées d'une partie externe 26 d'une pièce de liaison 1 et les extrémités arrière 27, 28 des deux branches sont destinées à être pincées de façon à être rapprochées l'une de l'autre manuellement afin d'écarter les extrémités avant pour obtenir manuellement la libération de la pièce de liaison 1. Tant que l'on n'exerce aucune action manuelle sur les branches 21, 22, la pince 20 reste serrée sur la partie supérieure 26 de la pièce de liaison 1 et peut ainsi être montée à demeure sur la pièce de liaison 1 à l'intérieur de l'enveloppe étanche 11 du dispositif de conditionnement 4, comme cela est représenté sur les figures 1D à 1G.

Les figures 3 et 4 représentent une variante de réalisation d'une pince spécifique 20'. La pince spécifique 20' comprend deux branches 31, 32 reliées l'une à l'autre par une articulation 33 et un dispositif de serrage par vis et écrou 34, 35. Quand la vis 34 est vissée dans l'écrou 35, des flancs coniques de la vis 34 tendent à pousser vers l'extérieur les extrémités arrière 36, 37 des branches 31, 32 tandis que les extrémités avant 38, 39 de ces mêmes branches tendent par conséquent à se rapprocher pour venir serrer la partie supérieure 26 de la pièce de liaison 1. La pince spécifique 20' peut être libérée de la pièce de liaison 1 en dévissant la vis 34 de façon à autoriser le rapprochement des extrémités arrière 36, 37 des branches 31, 32.

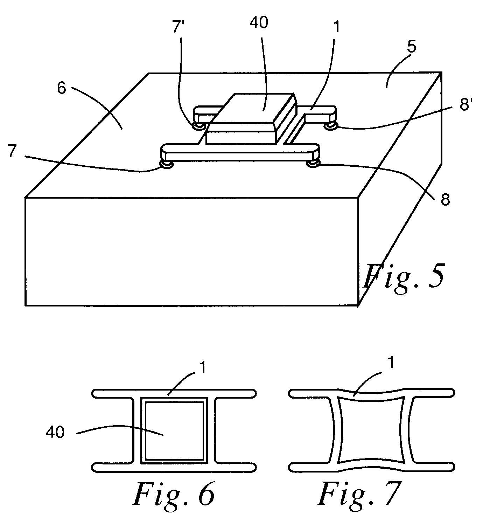

Le dispositif de conditionnement selon la présente invention peut être adapté pour permettre le conditionnement d'un grand nombre de pièces de liaison différentes. Par exemple, comme cela est représenté sur la figure 5, le dispositif de conditionnement peut comprendre une pièce de support 5 (décrite précédemment) comportant une face supérieure plane 6 et quatre logements 7, 8, 7', 8' débouchant au niveau de cette face supérieure 6. La pièce de liaison 1 adaptée à un tel dispositif est une pièce de liaison en forme de H qui comporte quatre éléments d'ancrage situés aux quatre coins d'une forme rectangulaire et qui sont destinés à venir s'enfiler respectivement dans les quatre logements 7, 8, 7', 8' de la pièce de support 5. La pièce de liaison 1 est représentée en vue de dessus sur les figures 6 et 7 de façon à montrer comment les différentes branches de liaison centrale de cette pièce peuvent s'incurver lors du réchauffement de la pièce (qui est en matériau à mémoire de forme) afin de provoquer un rapprochement des quatre éléments d'ancrage situés aux quatre coins de cette pièce. Cette pièce spécifique 1 n'est pas décrite plus en détail parce qu'elle est connue de l'art antérieur.

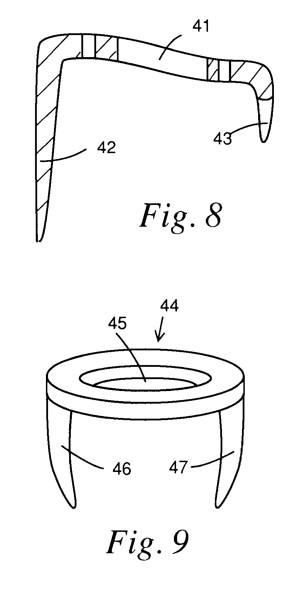

La figure 8 représente une agrafe de genou 41 qui constitue un autre mode de réalisation spécifique d'une pièce de liaison pour éléments osseux à matériau à mémoire de forme 1 utilisée dans le procédé et dans le dispositif de conditionnement selon la présente invention. Cette agrafe de genou 41 comprend deux extrémités opposées 42, 43 qui peuvent être pareillement fixées dans des logements spécifiques adaptés 7, 8 de la pièce de support 5 du dispositif de conditionnement 4 selon l'invention.

La figure 9 représente encore une autre pièce de liaison 44 pour éléments osseux incluant un matériau à mémoire de forme qui peut être avantageusement utilisée avec le dispositif de conditionnement 4 selon la présente invention. Cette autre pièce de liaison 44 comprend un trou central 45 et des éléments d'ancrage latéraux 46, 47. La déformation du trou central 45 permet aux éléments d'ancrage 46, 47 de se rapprocher l'un de l'autre afin d'améliorer la condition d'ancrage lors de la mise en place de la pièce de liaison.

L'invention n'est pas limitée aux modes de réalisation qui ont été décrits ici uniquement à titre d'exemples non limitatifs, mais l'invention s'étend à toutes les variantes imaginables qui restent dans l'esprit et dans le cadre général de l'invention.

## Revendications

1. Dispositif pour la liaison d'éléments osseux, caractérisé en ce qu'il comprend :
une pièce de liaison (1) comprenant une partie centrale et au moins deux parties d'ancrage (2, 3) liées rigidement à la partie centrale, au moins une partie de la pièce étant réalisée en un matériau à mémoire de forme ; et
un appareil de conditionnement comprenant un support (5) incluant des logements (7, 8) adaptés pour recevoir respectivement lesdites parties d'ancrage (3, 4) lorsque ces dites parties d'ancrage sont dans des positions d'éloignement maximum les unes par rapport aux autres.

2. Dispositif pour la liaison d'éléments osseux selon la revendication 1, caractérisé en ce que ladite partie centrale présente une forme générale d'un anneau circulaire ou ovale (45).

3. Dispositif pour la liaison d'éléments osseux selon la revendication 1, caractérisé en ce que ladite pièce de liaison est une plaque de genou (41) comprenant une partie centrale et au moins deux parties d'ancrage (42, 43).

4. Dispositif pour la liaison d'éléments osseux selon l'une quelconque des revendications précédentes, caractérisé en ce que ledit appareil de conditionnement comprend en outre un réservoir (12) renfermant de façon étanche un produit de chaleur massique élevée (15) de telle sorte qu'après que l'appareil de conditionnement a été refroidi puis placé en attente d'utilisation chirurgicale en un lieu à température ambiante, sa température reste basse pendant un temps relativement long, suffisant pour permettre à un chirurgien de choisir librement au cours d'une opération l'instant où il souhaite effectivement prélever la pièce de liaison placée dans l'appareil de conditionnement.
